# EUROPEAN PATENT SPECIFICATION

(11) **EP 4 189 117 B1**
(45) Date of publication and mention of the grant of the patent: **19.02.2025**
(21) Application number: 21758610.6
(22) Date of filing: 27.07.2021
(51) Int. Cl.: C12Q 1/6883

(54) **USE OF CIRCULAR RNA FOR THE DIAGNOSIS OF MULTIPLE SCLEROSIS**
VERWENDUNG VON ZIRKULÄRER RNA ZUR DIAGNOSE VON MULTIPLER SKLEROSE
UTILISATION D'ARN CIRCULAIRE POUR LE DIAGNOSTIC DE SCLÉROSE MULTIPLE

(30) Priority: 28.07.2020 ES 202030782
(43) Date of publication of application: 07.06.2023
(73) Proprietor: Administración General de la Comunidad Autónoma de Euskadi, 01010 Vitoria-Gasteiz, Álava (ES)
(72) Inventor: IPARRAGUIRRE GIL, Leire, 01010 Vitoria-Gasteiz (Álava) (ES); MUÑOZ CULLA, Maider, 01010 Vitoria-Gasteiz (Álava) (ES); CASTILLO TRIVIÑO, Tamara, 01010 Vitoria-Gasteiz (Álava) (ES); OTAEGUI BICHOT, David, 01010 Vitoria-Gasteiz (Álava) (ES)
(74) Representative: Pons
(86) International application number: PCT/EP2021/070958
(87) International publication number: WO 2022/023317

(56) References cited:
- WO-A2-2004/078098
- WO-A2-2016/172126
- IPARRAGUIRRE LEIRE ET AL: "Circular RNA profiling reveals that circular RNAs from ANXA2 can be used as new biomarkers for multiple sclerosis", HUMAN MOLECULAR GENETICS, vol. 26, no. 18, 15 September 2017 (2017-09-15), GB, pages 3564 - 3572, XP055834424, ISSN: 0964-6906, DOI: 10.1093/hmg/ddx243
- ZURAWSKA ANNA ET AL: "Circular RNAs as a novel layer of regulatory mechanism in multiple sclerosis", JOURNAL OF NEUROIMMUNOLOGY, vol. 334, 1 September 2019 (2019-09-01), NL, pages 576971, XP055859996, ISSN: 0165-5728, DOI: 10.1016/j.jneuroim.2019.576971
- IPARRAGUIRRE LEIRE ET AL: "RNA-Seq profiling of leukocytes reveals a sex-dependent global circular RNA upregulation in multiple sclerosis and 6 candidate biomarkers", HUMAN MOLECULAR GENETICS, vol. 29, no. 20, 18 December 2020 (2020-12-18), GB, pages 3361 - 3372, XP055860001, ISSN: 0964-6906, Retrieved from the Internet <URL:https://academic.oup.com/hmg/article-pdf/29/20/3361/34950025/ddaa219.pdf> DOI: 10.1093/hmg/ddaa219

## Description

The present invention relates to the use of a circular RNA of SEQ ID NO: 1 alone or in combination with other circular RNAs, as a biomarker(s) to diagnose multiple sclerosis in an effective, fast and reliable way.

### BACKGROUND OF THE INVENTION

Multiple sclerosis (MS) is an autoimmune demyelinating disease that affects more than 2.5 million people worldwide. MS is the second leading cause of disability in the population group between 20 and 40 years old; in particular, it is 3 times more prevalent in females than in males. MS represents a high cost for the healthcare system mainly due to its treatments, the chronicity of the disease and the disability it causes.

It has a complex pathophysiology with genetic and environmental factors that contribute to the development of the disease, and different clinical forms can be distinguished. For most patients (about 85%), the early phase of the disease (relapsing-remitting MS or RR-MS) is characterised by clinical exacerbations or relapses caused by autoreactive immune cells entering the central nervous system (CNS), thus resulting in focal inflammation and demyelination leading to symptoms of neurological disability that last at least 24 hours. After these relapses, the inflammation goes down and partial remyelination occurs, entering a recovery phase called remission. Over time, many patients start to have an incomplete recovery, leading to increased disability with little relapse. This phase of the disease is considered a second subtype called secondary progressive MS (SP-MS). For another 10-15% of patients, the disease progresses from the onset and is known as primary progressive or PP-MS (Compston A., et al., 2008. Multiple sclerosis. Lancet 372, 1502-1517*).*

The diagnosis of MS commonly involves finding clinical evidence of demyelinating lesions in the CNS, including the brain, the spinal cord and the optic nerves. Moreover, the lesions must spread in time and space. This clinical evidence can be based on the symptoms of patients, signs revealed by physical examination or different diagnostic tests. The most common diagnostic tests are magnetic resonance imaging (MRI) to locate lesions and the cerebrospinal fluid (CSF) test to confirm the presence of oligoclonal bands that suggest intrathecal IgG synthesis (Polman, C. H. et al. 2010, Diagnostic Criteria for Multiple Sclerosis. Revisions to the McDonald Criteria, 2011, Ann. Neurol. 69, 292-302*).*

Taking into account that MS affects the CNS, CSF is the most direct source of biomarkers; however, it is not an ideal source for biomarkers to be used continuously since multiple lumbar punctures are not recommended due to their invasiveness and potential adverse effects. Therefore, significant effort has been made to discover minimally invasive diagnostic biomarkers, such as blood biomarkers.

With this objective, and thanks to the arrival of omics techniques, different approaches have been carried out, one of them being gene expression profiling that, in addition to discovering new biomarkers, also makes it possible to elucidate the molecular mechanisms underlying the inflammatory responses in the disease. Dysregulation of both coding and non-coding RNAs (such as miRNA) and a clear gender bias have been demonstrated (Muñoz-Culla, M. et al. 2016, SncRNA (microRNA & snoRNA) opposite expression pattern found in multiple sclerosis relapse and remission is sex dependent. Sci. Rep. 6). Some of these transcriptions have been suggested as biomarkers for MS, but none of them have reached clinical practice.

Non-coding RNAs, such as miRNAs, are considered participants in the regulation of various immune responses and, as such, they have been proposed not only as targets of the disease but also as biomarkers, although very few have reached the clinical field. Circular RNAs (circRNAs) have recently emerged as new members of the non-coding RNA family and have been considered to be good non-invasive biomarkers in various diseases due to their high stability in body fluids (Zhang, Z., et al., 2018, Circular RNAs: Promising Biomarkers for Human Diseases. EBioMedicine 34, 267-274*).*

In recent years, circRNAs have emerged as new players in the scope of RNA, with an important role in post-transcriptional regulatory processes. They have been found to participate in various processes, such as in tumours, metabolism and immune pathways. As a result, they have also been linked to various diseases, including cancers, cardiovascular diseases, neurological diseases and autoimmune diseases (Aufiero, S., et al., 2019, Circular RNAs open a new chapter in cardiovascular biology. Nat. Rev. Cardiol. 16, 503-514*) (*Xia, X., et al., 2019, Roles of CircRNAs in Autoimmune Diseases. Front. Immunol. 10, 1-8*).*

IPARRAGUIRRE LEIRE ET AL, ,HUMAN MOLECULAR GENETICS, vol. 26, no. 18, 15 September 2017 (2017-09-15), pages 3564-3572, refers to circular RNAs from ANXA2 as new biomarkers for multiple sclerosis. However, both the course of the disease and the clinical phenotype of MS widely vary among patients and within the same individual. Currently, there are no effective short-term biomarkers and most of the time the sample must be cerebrospinal fluid. For this reason, it is necessary to develop alternative biomarkers to those that already exist in the state of the art which can help with the early, viable diagnosis of MS, preferably samples that can be obtained easily such as blood.

### DESCRIPTION OF THE INVENTION

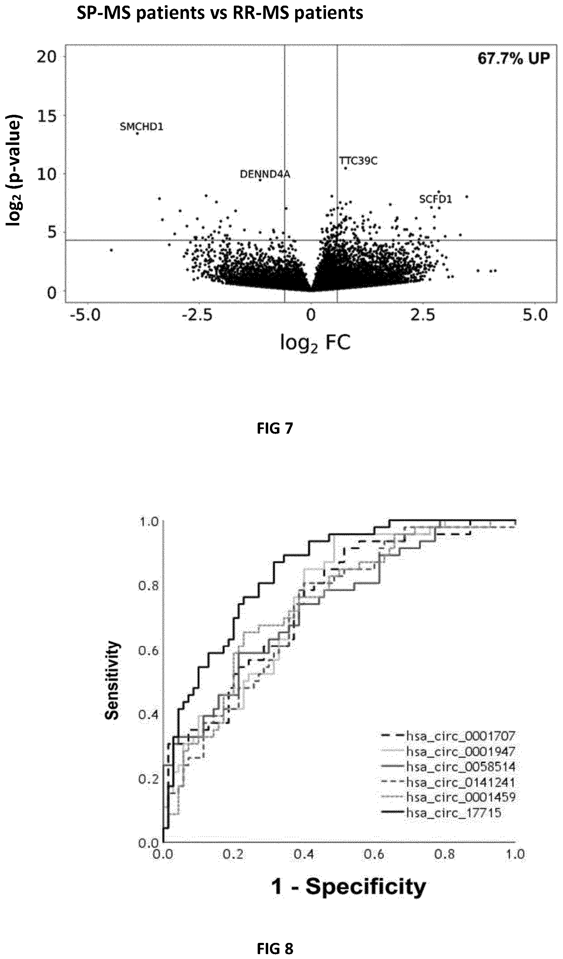

The present invention is defined by the appended claims. The inventors have demonstrated the potential of a set of circular RNAs (circRNA) as biomarkers for the diagnosis of MS. Using RNA-Seq, they analysed the profile of circRNAs in blood samples from patients with MS and from healthy patients (healthy controls or HC), detecting an altered expression pattern of circRNAs in patients with MS compared to HC patients. After the analysis, they selected the best candidates based on their expression level in the change occurring in the expression between the two groups (Fold-Change) and in the p-value obtained in the corrected statistical test (FDR). Furthermore, in a second cohort of 84 patients with MS and 51 healthy controls, they validated the best circRNA candidates and obtained 6 significantly upregulated circRNAs, the circRNAs: circRNA_PADI4 [1] (hsa_circ_17715), circRNA_0001707 [2], circRNA_0001947 [3], circRNA_0058514 [4], circRNA_0141241 [5] and circRNA_1459 [6], and therefore, they had a better outcome as biomarkers of MS, circRNA_PADI4 [1] being the biomarker with the best results, with an area under the curve value of 0.843 (see Table 5 and Figure 8 herein).

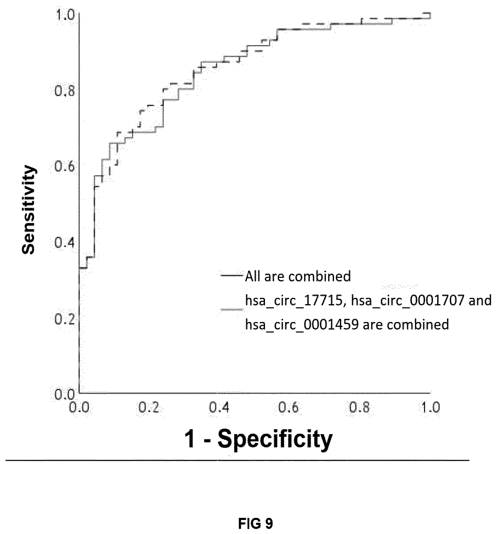

Finally, the inventors analysed the possibility of combining several circRNAs to improve the predictive ability of the markers. They observed that the combination of circRNA_PADI4 (hsa_circ_17715) [1] with the circRNAs [2] and [6] improves the area under the curve until a value of 0.847 is obtained, whereas the combination of the 6 validated circRNAs is the one that enables the maximum area under the curve value to be obtained, 0.853 as can be seen herein in Figure 9 or Table 5.

Therefore, the inventors have demonstrated the usefulness of circRNA_PADI4[1] as a diagnostic biomarker of MS in an efficient, fast and reliable way, and that said efficiency and reliability is improved when said circRNA is combined with other circRNAs, particularly when the six circRNAs are combined. The *circRNADb* Database access number for said PADI4 circRNA [1] is *Circ ID: hsa_circ_17715,* which belongs to the PADI4 gene and comprises the nucleotide sequence SEQ ID NO: 1.

Therefore, in a first aspect, the present invention relates to the *in vitro* use of a circRNA comprising the sequence SEQ ID NO: 1 as a biomarker for the diagnosis of multiple sclerosis (MS).

In the present invention, "diagnosis" or "diagnose" is understood as the process or act of recognising, determining or drawing conclusions about a disease or condition in a subject, based on the symptoms, signs and/or results of different variables, such as, for example, knowing the presence, absence, quantity and/or expression levels of one or more biomarkers characteristic of the disease or condition to be diagnosed. In the present invention, the "diagnosis of multiple sclerosis" or "diagnosis of MS" in a subject means, in particular, that the subject suffers from multiple sclerosis.

In the present invention, MS is diagnosed by using a circRNA as a biomarker. The term "biomarker" refers to a molecule or the expression product of a gene or fragments and variants thereof that show substantial changes in a given disease and that can be used both for the detection and for the diagnosis of a disease by detecting the appearance of said changes in the biomarker and/or for monitoring the efficacy of a treatment for that disease.

The biomarker for diagnosing MS is a circRNA. The term "circular RNA" or "circRNA", used interchangeably herein, refers to a type of single-stranded RNA that, unlike linear RNA, forms a covalently closed continuous loop; in other words, in the circRNA the 3'and 5' ends present in an RNA molecule have been joined. It is routine practice for a person skilled in the art to identify the circRNAs of the state of the art described in documents or databases.

Examples of circRNA databases include, without limitation, "circBase" (http://www. circbase.org/), "CircNet" (https://omictools.com/circnet-tool), "circRNABase" (http://starbase.sysu.edu.cn/starbase2/mirCircRNA.php), "circRNADb - A database for human circular RNAs" (http://reprod.njmu.edu.cn/cgi-bin/circrnadb/circRNADb.php) or "Circ2Traits" (http://gyanxet-beta.com/circdb/).

The inventors observed that the circRNA "hsa_circ_17715" (*circRNADb*) comprising the nucleotide sequence SEQ ID NO: 1, hereinafter the "circRNA of the invention", is differentially overexpressed in patients with MS compared to healthy patients, in other words, those who do not suffer from MS, this circRNA being a biomarker for the diagnosis of MS.

As understood by a person skilled in the art, the term circRNA of the invention or circRNA hsa_circ_17715 contemplates all those variants that, even having different nucleotide sequences due to neutral nucleotide mutations (conservative substitutions), perform the same function as said circRNA in humans. Thus, in a particular embodiment of the invention, the gene encoding the circRNA of the invention comprises a nucleotide sequence with a sequence identity of, at least, a 60, 65, 70, 75, 80, 81, 82, 83, 84, 85, 86, 87, 88, 89, 90, 91, 92, 93, 94, 95, 96, 97, 98 or 99% identity with the sequence SEQ ID NO: 1. The degree of identity is determined using computer algorithms and procedures that are widely known to the person skilled in the art. The identity between two amino acid/nucleotide sequences is preferably determined using the BLASTP/BLASTN algorithm [BLAST Manual, Altschul, S., et al., NCBI NLM NIH Bethesda, Md.20894, Altschul, S., et al., J. Mol. Biol. 215:403-410 (1990)]. In another more particular embodiment, the circRNA of the invention comprises a nucleotide sequence with 100% identity with the sequence SEQ ID NO: 1. In another even more particular embodiment, the circRNA of the invention consists of the sequence SEQ ID NO: 1.

The circRNA of the invention is differentially overexpressed in patients with MS; therefore, the expression levels of said circRNA can be used as a biomarker to diagnose MS. Therefore, another particular embodiment of the present invention relates to the *in vitro* use of the expression levels of a circular RNA comprising the sequence SEQ ID NO: 1 as a biomarker for the diagnosis of multiple sclerosis (MS).

The term "expression", as used herein, refers to the process by means of which an RNA molecule is produced from a sequence in the DNA genome in the process called transcription. In the context of the invention, "expression levels" refers to any quantifiable change in RNA expression or production, which produces altered relative levels of RNA in a sample relative to other molecules in the same sample. It will be seen that the expression levels of a biomarker can be determined by determining the RNA levels.

As understood by a person skilled in the art, the evaluation and determination of the expression levels of the circRNA of the invention for the diagnosis of MS requires a statistically significant result from the subjects so that it can be identified as a carrier of the disease. A person skilled in the art is able to determine whether the expression levels of the circRNA in a subject with MS is statistically significant relative to a healthy subject by using statistical evaluation tools. Examples of statistical tools are, without limitation, determination of confidence intervals, determination of p-values, Student's t test or Mann-Whitney test. In a particular embodiment, the p-values are preferably less than 0.1, more preferably a p-value less than 0.05.

As understood by a person skilled in the art and as previously described herein, the term circRNA of the invention contemplates all those variants that, even having different nucleotide sequences, perform the same function as the circRNA of the invention. Likewise, the overexpression of said variants may indicate that the subject suffers from MS and, therefore, the expression levels thereof can be used as biomarkers for the diagnosis of MS.

Thus, disclosed, but not part of the claimed invention is the *in vitro* use of the expression levels of a circRNA comprising a nucleotide sequence with a sequence identity of, at least, a 60, 65, 70, 75, 80, 81, 82, 83, 84, 85, 86, 87, 88, 89, 90, 91, 92, 93, 94, 95, 96, 97, 98 or 99% identity with the sequence SEQ ID NO: 1 as a biomarker for the diagnosis of multiple sclerosis (MS). The degree of identity is determined using computer algorithms and procedures that are widely known to the person skilled in the art and are previously described herein. Another more particular embodiment relates to the *in vitro* use of the expression levels of a circRNA comprising a nucleotide sequence with 100% identity with the sequence SEQ ID NO: 1.

Another even more particular embodiment relates to the *in vitro* use of the expression levels of a circRNA consisting of the sequence SEQ ID NO: 1.

The inventors also observed other differentially overexpressed circRNAs in patients with MS. Access numbers or identifiers (*circ RNA ID*) from the *circBase* database of said circRNAs are the following: hsa_circ_0001707 (SEQ ID NO: 2), hsa_circ_0001947 (SEQ ID NO: 3), hsa_circ_0058514 (SEQ ID NO: 4), hsa_circ_0141241 (SEQ ID NO: 5) and hsa_circ_0001459 (SEQ ID NO: 6). The inventors analysed the combination of these circRNAs with the circRNA of the invention and obtained an improvement in the predictive ability of the diagnosis of MS.

Therefore, another particular embodiment of the invention relates to the *in vitro* use of the circRNA of the invention in combination with at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6, as a biomarker for the diagnosis of multiple sclerosis (MS).

The terms "diagnosis of MS", "biomarker" and "circular RNA" have already been previously defined herein, and they likewise apply to this embodiment of the invention.

As understood by a person skilled in the art, this set of circRNAs for the combination thereof with the circRNA of the invention contemplates all those variants that, even having different nucleotide sequences, perform the same function as hsa_circ_0001707 (SEQ ID NO: 2), hsa_circ_0001947 (SEQ ID NO: 3), hsa_circ_0058514 (SEQ ID NO: 4), hsa_circ_0141241 (SEQ ID NO: 5) and hsa_circ_0001459 (SEQ ID NO: 6). Thus, another particular embodiment of the invention relates to the *in vitro* use of the circRNA of the invention in combination with at least one, two, three, four or five of the aforementioned circRNAs, wherein the gene encoding each of these circRNAs comprises, or consists of, a nucleotide sequence with a sequence identity of, at least, a 60, 65, 70, 75, 80, 81, 82, 83, 84, 85, 86, 87, 88, 89, 90, 91, 92, 93, 94, 95, 96, 97, 98 or 99% identity with the sequence SEQ ID NO: 2, SEQ ID NO: 3, SEQ ID NO: 4, SEQ ID NO: 5 or SEQ ID NO: 6.

Another more particular embodiment of the invention relates to the *in vitro* use of the circRNA of the invention in combination with at least one, two, three, four or five of the aforementioned circRNAs, wherein the gene encoding each of these circRNAs comprises a nucleotide sequence with 100% identity with the sequence SEQ ID NO: 2, SEQ ID NO: 3, SEQ ID NO: 4, SEQ ID NO: 5 or SEQ ID NO: 6.

Another even more particular embodiment of the invention relates to the *in vitro* use of the circRNA of the invention in combination with at least one, two, three, four or five of the aforementioned circRNAs, wherein the gene encoding each of these circRNAs consists of the sequence SEQ ID NO: 2, SEQ ID NO: 3, SEQ ID NO: 4, SEQ ID NO: 5 or SEQ ID NO: 6. These circRNAs are overexpressed in patients with MS; therefore, said expression levels can be used as biomarkers to diagnose MS in combination with the expression levels of the circRNA of the invention.

Therefore, another particular embodiment of the present invention relates to the *in vitro* use of the expression levels of the circRNA of the invention in combination with the expression levels of at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6, as a biomarker for the diagnosis of multiple sclerosis (MS).

The term "expression levels" has already been defined herein, and it likewise applies to this particular embodiment of the invention.

As understood by a person skilled in the art, and as previously described herein, this set of circRNAs for the combination thereof with the circRNA of the invention contemplates all those variants that, even having different nucleotide sequences, perform the same function as hsa_circ_0001707 (SEQ ID NO: 2), hsa_circ_0001947 (SEQ ID NO: 3), hsa_circ_0058514 (SEQ ID NO: 4), hsa_circ_0141241 (SEQ ID NO: 5) and hsa_circ_0001459 (SEQ ID NO: 6), and likewise, a high expression level of said variants relative to a reference value may indicate that the subject suffers from MS, and therefore, it may be used together with the expression levels of the circRNA of the invention as biomarkers for the diagnosis of MS.

Thus, another particular embodiment of the invention relates to the *in vitro* use of the expression levels of the circRNA of the invention in combination with the expression levels of at least one, two, three, four or five of the circRNAs, wherein the gene encoding each of these circRNAs comprises a nucleotide sequence with a sequence identity of, at least, a 60, 65, 70, 75, 80, 81, 82, 83, 84, 85, 86, 87, 88, 89, 90, 91, 92, 93, 94, 95, 96, 97, 98 or 99% identity with the sequence SEQ ID NO: 2, SEQ ID NO: 3, SEQ ID NO: 4, SEQ ID NO: 5 or SEQ ID NO: 6.

Another more particular embodiment of the invention relates to the *in vitro* use of the expression levels of the circRNA of the invention in combination with the expression levels of at least one, two, three, four or five of the circRNAs, wherein the gene encoding each of these circRNAs comprises a nucleotide sequence with 100% identity with the sequence SEQ ID NO: 2, SEQ ID NO: 3, SEQ ID NO: 4, SEQ ID NO: 5 or SEQ ID NO: 6.

Another even more particular embodiment of the invention relates to the *in vitro* use of the expression levels of the circRNA of the invention in combination with the expression levels of at least one, two, three, four or five or six of the circRNAs, wherein the gene encoding each of these circRNAs consists of the sequence SEQ ID NO: 2, SEQ ID NO: 3, SEQ ID NO: 4, SEQ ID NO: 5 or SEQ ID NO: 6.

The inventors observed that by combining the circRNA of the invention, hsa_circ_17715 (SEQ ID NO:1), together with the other 5 validated circRNAs, the best predictive results were obtained for the diagnosis of MS, shown herein in Figure 9.

Therefore, another more particular embodiment of the present invention relates to the *in vitro* use of the circRNA of the invention (SEQ ID NO: 1), in combination with the 5 validated circRNAs comprising the sequences (SEQ ID NO: 2), (SEQ ID NO:3), (SEQ ID NO:4), (SEQ ID NO:5), (SEQ ID NO:6).

These circRNAs are overexpressed in patients with MS; therefore, the combination of the expression levels of said circRNAs can also be used to diagnose MS. As such, another particular embodiment of the present invention relates to the *in vitro* use of the expression level of the circRNA of the invention in combination with the 5 validated circRNAs comprising the sequences (SEQ ID NO: 2), (SEQ ID NO:3), (SEQ ID NO:4), (SEQ ID NO:5), (SEQ ID NO:6).

As previously described herein, the inventors demonstrated that the circRNA of the invention, as well as the expression levels thereof, both individually and in combination with other circRNAs, allow for an *in vitro* diagnosis of MS in a subject.

Therefore, another aspect of the present invention relates to an *in vitro* method for the diagnosis of multiple sclerosis, hereinafter the "method of the invention", comprising:
(a) determining the expression of the circRNA comprising the sequence SEQ ID NO: 1 in an isolated sample from a subject, and
(b) comparing it with a reference value, wherein an increase in the expression of said circRNA relative to the reference value indicates that the subject suffers from MS.

The terms "diagnosis of MS", "circular RNA" or "expression level" have already been previously described herein and likewise apply to this inventive aspect, as well as to all the particular embodiments thereof.

Step (a) of the method of the invention requires determining the expression of the circRNA of the invention in an isolated sample from the subject.

The term "isolated sample", as used in the present invention, refers to any sample obtained or coming from an individual which comprises the nucleic acid, in other words, the genetic material of living organisms that controls inheritance and is located in the nucleus of the cells. Examples of biological samples include, without limitation, biopsy, tissue, solid stool and biofluids, such as urine, faeces, serum, saliva, semen, sputum, cerebrospinal fluid (CSF), tears, mucus, sweat, milk, brain extracts and the like. In a particular embodiment of the method of the invention, the biological sample is blood.

The biological sample is isolated from a subject. As used in the present invention, the term "subject" or "individual" refers to any animal that has a nervous system, specifically, a central nervous system. Preferably, the subject is a mammal, more preferably a human. The term does not indicate a particular age or gender. In a particular embodiment, the subject of the method of the invention is a mammal, preferably a primate, more preferably a human being of any gender or age.

Once the biological sample isolated from the subject has been obtained, the expression level of the circRNA must be determined, step (a) of the method of the invention. It is routine practice for a person skilled in the art to determine the expression level of a gene, particularly the expression level of a circRNA, by using techniques widely known in the state of the art. Examples of techniques and methods for determining expression levels include, without limitation, hybridisation-based methods such as Northern blot analysis and in situ hybridisation, methods combining hybridisation with flow cytometry, hybridisation assays on arrays such as microarrays or NanoString, PCR methods such as quantitative PCR (qPCR) or digital droplet PCR (ddPCR) either by means of assays based on Taqman probes or based on the detection of SYBR Green, and large-scale sequencing methods such as RNA-Seq.

Thus, in a particular embodiment of the method of the invention, the determination of the expression of the circRNA is determined by means of RNA-Seq, quantitative PCR (qPCR) or digital PCR (ddPCR).

Later, once the expression level of the circRNA of the invention in the subject has been obtained, it must be statistically compared to a reference value, wherein an increase in the expression of said circRNA relative to the reference value indicates that the subject suffers from MS.

The term "reference level" or "reference value" herein used interchangeably refers to a predetermined criterion used as a reference to evaluate the values or data obtained from samples collected from a subject. The reference value or reference level can be an absolute value, a relative value, a value that has an upper or lower limit, a range of values, an average value, a median value, a mean value or a value compared to a particular control. The reference value can be based on an individual sample value, such as, for example, a value obtained from a sample of the subject who is being evaluated, but at an earlier time. Also, the reference value can be based on a large number of samples, such as the population of subjects in the chronological age group, or based on a group of samples that include or exclude the sample to be analysed.

In another particular embodiment of the present invention, the reference level is the expression level of the circRNA of the invention in a healthy subject, in other words, an individual who does not suffer from MS.

A person skilled in the art is able to determine whether the expression level of the circRNA in a subject with MS is statistically significant relative to the reference value (expression level of the circRNA in a healthy subject) by using statistical evaluation tools. Examples of statistical tools are, without limitation, determination of confidence intervals, determination of p-values, Student's t test or Mann-Whitney test. In a particular embodiment, the p-values are preferably less than 0.1, more preferably a p-value less than 0.05.

Moreover, as previously described herein, the inventors also observed that the determination of the expression levels of the circRNA of the invention in combination with the determination of the expression levels of other circRNAs made it possible to diagnose MS in a subject with greater predictive ability. These circRNAs, like in the previous aspect of the present invention, have the following Circ IDs from the *circBase* database: hsa_circ_0001707 (SEQ ID NO: 2), hsa_circ_0001947 (SEQ ID NO: 3), hsa_circ_0058514 (SEQ ID NO: 4), hsa_circ_0141241 (SEQ ID NO: 5) and hsa_circ_0001459 (SEQ ID NO: 6).

As such, another particular embodiment of the method of the invention relates to an *in vitro* method which further comprises determining the expression of at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6, wherein an increase in the expression of said circRNA relative to the reference value indicates that the subject suffers from MS.

The terms "diagnosis of MS", "circular RNA", "expression level" or "reference value" have already been previously described herein, and likewise apply to this particular embodiment, as well as to successive particular embodiments herein.

As previously described herein, the inventors observed that by determining the expression levels of the combination of the circRNA of the invention, hsa_circ_17715 (SEQ ID NO:1), together with the circRNAs hsa_circ_0001707 (SEQ ID NO: 2), hsa_circ_0001947 (SEQ ID NO: 3), hsa_circ_0058514 (SEQ ID NO: 4), hsa_circ_0141241 (SEQ ID NO: 5) and hsa_circ_0001459 (SEQ ID NO:6), the best predictive results were obtained for the diagnosis of MS.

Therefore, another more particular embodiment of the method of the invention relates to an *in vitro* method which comprises determining the expression of the circRNAs comprising the sequences SEQ ID NO: 1, 2, 3, 4, 5 and 6, wherein an increase in the expression of said circRNAs relative to the reference value indicates that the subject suffers from MS.

The implementation of the method of the invention comprises the use of a set of reagents that can be comprised within a kit.

Therefore, in another aspect, the present invention relates to use of a kit that comprises the reagents needed to quantify the expression of the circRNA comprising the sequence SEQ ID NO: 1, hereinafter "kit of the invention", wherein said reagents comprise:
- a primer pair that specifically hybridises to the nucleotide sequence of the circRNA, and/or
- a probe that specifically hybridises to the nucleotide sequence of the circRNA, for measuring the expression of SEQ ID NO: 1, to diagnose MS.

In the present invention, "kit" is understood as a product that comprises the different reagents needed to carry out the methods of the invention adapted to enable said reagents to be transported and stored. Suitable materials for the packaging of the components of the kit can be plastic (such as polyethylene, polypropylene, polycarbonate and the like), or glass, and they can comprise bottles, jars, envelopes, etc. Moreover, the kit of the invention may contain instructions for simultaneous, sequential or separate use of the different components found in the kit. Such instructions may be provided as printed material, or as an electronic medium capable of storing instructions, such as storage disks (magnetic disks), optical methods (CD-ROM, DVD, etc.) and the like.

The expression "reagents needed to quantify the expression levels of the circular RNA" means a compound or group of compounds that enable the expression level of an RNA sequence to be determined. Examples of reagents needed to quantify the expression levels of the circRNA include, without limitation, a buffer, mixtures of deoxyribonucleotide triphosphates (dNTPs), reverse transcriptase, RNAase inhibitors, conventional or real-time thermal cycler and fluorophore probes (such as SYBR Green).

Moreover, the kit can comprise the reagents needed to quantify the expression level of a combination of circRNAs, wherein said circRNAs have already been previously described herein and are the same for this inventive aspect. Therefore, the kit of the invention can further comprise primer pairs of said circRNAs that are combined with the circRNA of the invention, the sequences of which are herein described in Table 2.

Therefore, in another particular embodiment, the kit further comprises the reagents to quantify the expression of at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6.

Once the reagents are comprised within the kit, this kit is used to implement the method of the invention.

Thus, another aspect of the present invention relates to the *in vitro* use of the kit of the invention for the diagnosis of MS.

The terms "kit" and "diagnosis of MS" have already been previously described herein and are applicable to this inventive aspect.

Throughout the description and the claims, the word "comprises" and its variants are not intended to exclude other technical features, additives, components or steps. For those skilled in the art, other objects, advantages and features of the invention will be apparent in part from the description and in part from the practice of the invention. The following examples and figures are provided by way of illustration and are not intended to limit the present invention.

### BRIEF DESCRIPTION OF THE FIGURES

**Figure 1****.** Diagram showing the RNA-Seq workflow from the preparation of samples to differential expression.
**Figure 2****.** General features of the circRNA profile. A) Correlation of the quantification performed by find_circ and Ciri2. B) Histogram showing the number of reads per circRNA. C) Overall expression of circRNA for each sample. D) circRNA distribution in chromosomes. E) Number of circRNAs produced from each gene.

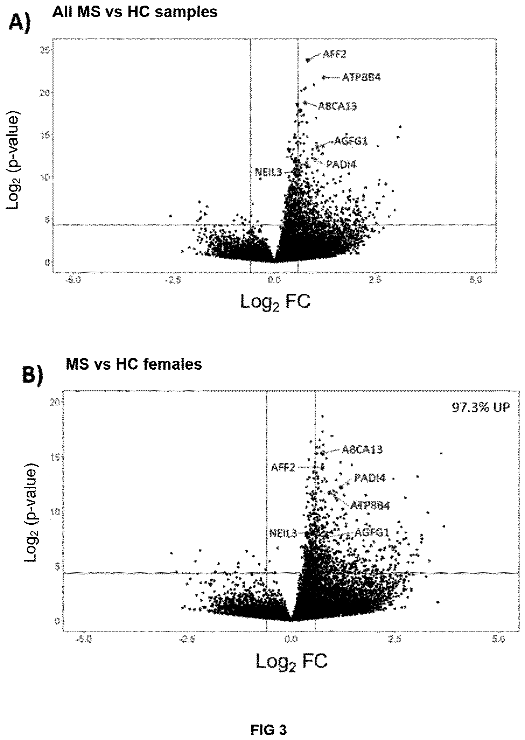

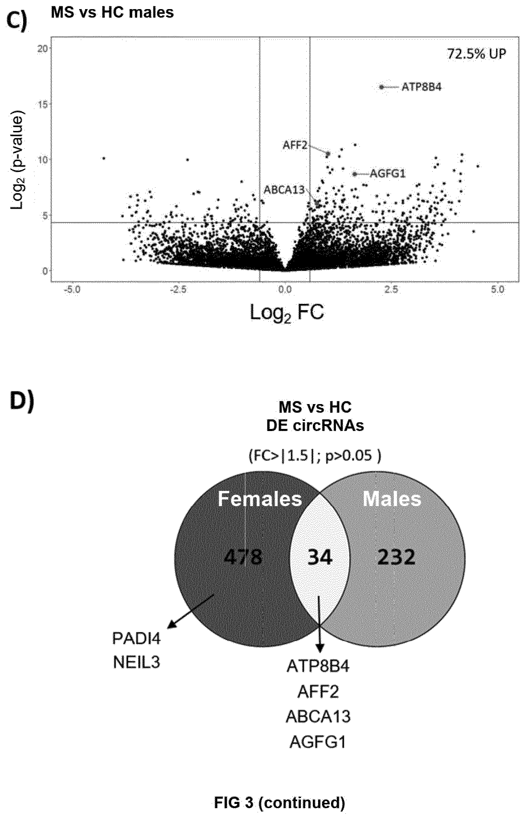

**Figure 3.**464 differentially expressed circRNAs (DE-circRNA) that have been found (p-value <0.05, FC> 1.5) with a clear trend toward positive regulation in patients (446 upregulated and 18 downregulated) and a gender bias. It shows volcano plots showing DE-circRNA between A) all patients with MS and HC (30 versus 20), B) Female patients with MS and HC (23 versus 14), C) Male patients with MS and HC (7 versus 6). The ratio between the number of significantly upregulated circRNAs and downregulated circRNAs for each of the comparisons is shown in the upper left corner of each volcano plot. D) Venn diagram comparing the DE-circRNAs between genders showing that there is a gender-specific signature. 7 circRNAs selected for qPCR validation are indicated. Abbreviations: DE-circRNAs, differentially expressed circRNAs; MS, multiple sclerosis; HC, healthy controls.
**Figure 4****.** Detection of the amplicons of each circRNA in the agarose gel and the electropherograms showing the sequence corresponding to the BSJ of each circRNA.
**Figure 5.** 6 circRNA candidates that are upregulated in a second independent cohort (70 MS and 46 HC). The t-test was performed for statistical analysis. Abbreviations and symbols: ns, not significant; ****, p-value≤0.0001
**Figure 6****.** CircRNA overexpression is maintained when the analysis is limited to females. In males, only three circRNAs remain overexpressed. The Student's t-test was used for statistics. Abbreviations and symbols; HCF, Healthy Control Female; MSF, Multiple Sclerosis Female; HCM, Healthy Control Male; MSM, Multiple Sclerosis Male; ns, not significant; ****, p-value≤0.0001; ***, p-value≤0.001; **, p-value≤0.01; *, p-value≤0.05;
**Figure 7****.** Volcano plot of differentially expressed circRNAs between SP-MS patients (n = 10) and RR-MS patients (n = 20). The percentage of upregulated circRNAs is shown in the upper right quadrant of the volcano plot. Abbreviations: SP-MS, Secondary Progressive Multiple Sclerosis; RR-MS, Relapsing-Remitting Multiple Sclerosis; UP, Upregulated; FC, Fold Change.
**Figure 8****.** ROC curves for each of the 6 selected circRNAs that show the potential thereof to be biomarkers in multiple sclerosis.
**Figure 9****.** ROC curves showing the predictive value of combining the hsa_circ_17715 circRNA with two or more of the selected circRNAs.

### EXAMPLES

The invention is illustrated below by means of assays carried out by the inventors which reveal the effectiveness of the invention.

### MATERIALS AND METHODS

### Obtaining blood samples and extracting RNA

The inventors collected the whole blood of a total of 66 healthy controls (HC) and 100 patients with multiple sclerosis (MS) matched by age and gender at the Department of Neurology at the Hospital Universitario de Donostia. The erythrocytes were lysed with EL buffer (Qiagen) and the total RNA was isolated from the leukocytes with the miRNeasy Mini Kit (Qiagen) following the manufacturer's instructions. The inventors measured the RNA concentration using a NanoDrop ND-1000 spectrophotometer (Thermo Scientific).

The inventors divided the samples into two cohorts. The discovery cohort consisted of 20 HC and 30 patients with MS (20 RR-MS and 10 SP-MS) and they were analysed by RNA-Seq. Furthermore, 46 HC and 70 MS (62 RR-MS and 8 SP-MS) samples were included in the validation cohort and subjected to real-time PCR (RT-qPCR). The main clinical and demographic characteristics of the patients and healthy controls are summarised in Table 1. The inventors collected samples from all individuals after receiving their written informed consent. The study was approved by the hospital's ethics committee and the samples were processed and stored in the Basque Biobank (http://www.biobancovasco.org).

**Table 1. Main clinical and demographic characteristics of the individuals included in the study (100 patients with multiple sclerosis and 66 healthy controls in two different cohorts). Abbreviations: MS, Multiple Sclerosis. HC, Healthy Control. RR-MS, Relapsing-Remitting Multiple Sclerosis. SP-MS, Secondary Progressive Multiple Sclerosis. EDSS, Expanded Disability Status Scale. Time evo., Time of evolution. AOO, Age of onset. Age, Time evo. and AOO are presented as 'average (standard deviation)', EDSS is presented as 'median (range)'.**

| | | Gender | Age | Type of MS | EDSS | Time evo. | AOO |
|---|---|---|---|---|---|---|---|
| Discovery cohort | MS (n=30) | Female (n=23) | 44.3 (±10.0) | 16RR-MS/7SP-MS | 3.7 (0-7) | 14.7 (±10.4) | 29.6 (±7.4) |
| | | Male (n=7) | 44.7 (±8.5) | 4RR-MS/3SP-MS | 4.6 (2-8) | 15.7 (±7.6) | 29.0 (±10.1) |
| | HC (n=20) | Female (n=14) | 49.6 (±7.9) | - | - | - | - |
| | | Male (n=6) | 38.5 (±4.5) | - | - | - | - |
| Validation cohort | MS (n=70) | Female (n=58) | 41.3 (±9.5) | 50RR-MS/8SP-MS | 2.3 (0-8) | 10.2 (±7.6) | 31.1 (±8.9) |
| | | Male (n=12) | 40.0 (±8.2) | 12RR-MS | 2.4 (0-4) | 5.9 (±3.7) | 28.9 (±9.8) |
| | HC (n=46) | Female (n=14) | 33.2 (±8.7) | - | - | - | - |
| | | Male (n=18) | 34.9 (±10.2) | - | - | - | - |

### RNA-Seq

The inventors prepared the library and the next generation sequencing in CD Genomics (USA). They remeasured the concentration and quality of the RNA samples using the Bioanalyzer 2100 instrument (Agilent) prior to preparing the library. After standardisation, they removed the rRNA from the total RNA sample using the Ribo-Zero rRNA removal kit, followed by the purification and fragmentation steps.

To build the sequencing libraries, the inventors performed a strand-specific cDNA synthesis, the 3' ends were adenylated and the adapters were ligated. The resulting libraries were subjected to quality control and a standardisation process. They performed the sequencing using paired reads with Illumina HiSeq PE150 and obtained an average of 10 Gb of data per sample.

### CircRNA detection and quantification in RNA-Seq data

The inventors checked the sequencing reads for quality and mapped them against the GRCh37/hg19 version of the genome using the BWA or Bowtie aligners. Later, they detected circRNA by using the find_circ, version 1.0 and Ciri2 algorithms following the recommendation of the authors. For find_circ, they used a higher stringency threshold which requires both adapter sequences to be mapped with the highest mapping quality possible (mapq = 40). Moreover, only circRNAs detected with at least two reads in a given sample and found by both algorithms were used in subsequent analyses.

The expression level of circRNAs was based on the quantification of the number of reads spanning BSJ according to that of the Ciri2 algorithm. The differential expression analysis was performed using DESeq2 in R-studio.

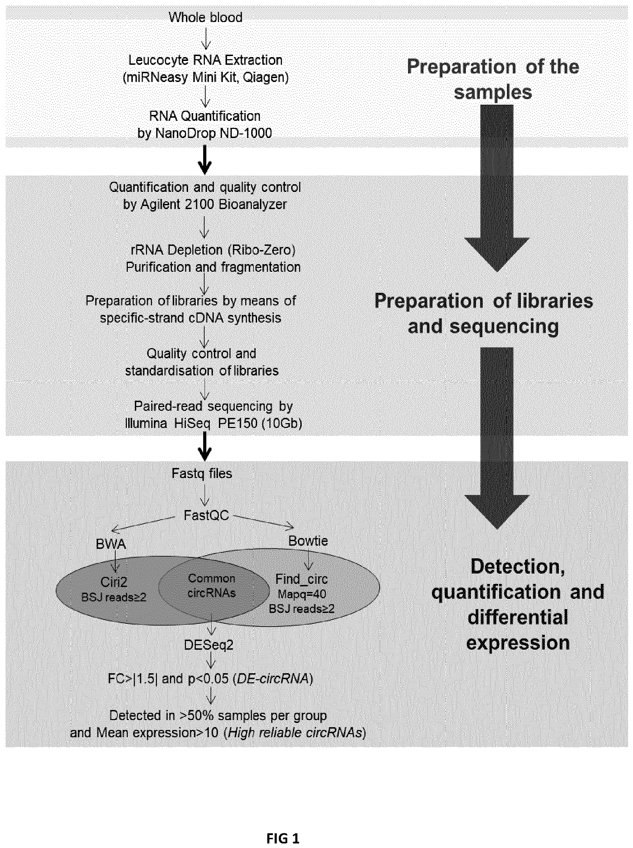

CircRNAs with a fold-change (FC) value greater than 1.5 (FC> 1.5) and a p-value less than 0.05 (p <0.05) were considered to be differentially expressed circRNA (hereinafter, DE-circRNA). The inventors applied additional screening criteria to these DE-circRNAs to select circRNA candidates for validation by RT-qPCR. The inventors selected these circRNA candidates from among the highly reliable DE-circRNAs defined as those detected in at least 50% of the samples in each group and with a mean expression value, defined as the mean of the standardised counts of all samples, greater than 10. All of these steps that are explained above are shown schematically in Figure 1, from the preparation of the sample to differential expression.

### cDNA synthesis and quantitative PCR

The inventors reverse transcribed 600 ng of total RNA into cDNA with random primers using the "High capacity" cDNA reverse transcription kit (Applied Biosystems, Inc., USA). RT-qPCR was performed in a Veriti thermal cycler (Applied Biosystems, Inc., USA) with the following programme: 25°C for 10 minutes, 37°C for 120 minutes and 85°C for 5 minutes. Quantitative PCR was performed using Power SYBR Green Master Mix (Applied Biosystems) on a CFX384 Touch Real-Time PCR Detection System (Bio-Rad laboratories, Inc.) following the manufacturer's instructions.

The inventors used divergent primers to amplify the circRNAs so that the amplicon spans the back-spliced junction and they used EEF1A1 as the reference gene for standardisation (Table 2).

**Table 2. Sequence of the primers used to amplify the circRNAs by RT-qPCR**

| | | | **Direct primer** | | **Reverse primer** | | **Product** |
|---|---|---|---|---|---|---|---|
| **Genomic location (GRCh37)** | **Gene** | **circBase ID** | **Sequence** | **Mt (°C)** | **Sequence** | **Mt (°C)** | **Size (Pb)** |
| chr7:48541721-48542148 | ABCA13 | hsa_circ_0001707 | ATTGAGCTCCCCACAGAA AC (SEQ ID NO: 7) | 59.1 | CTCGCTGGTGAAATGATT GA (SEQ ID NO: 8) | 59.8 | 187 |
| chrX:147743428-147744289 | AFF2 | hsa_circ_0001947 | TTTGGGAATCTGTCATTTG GA (SEQ ID NO: 9) | 60.3 | ACCTTTGTTTGTTTCACTT GTTTG (SEQ ID NO: 10) | 59.5 | 111 |
| chr15:50294349-50311173 | ATP8B4 | hsa_circ_0141241 | TGAGCCACATGGTCTCTG TT (SEQ ID NO: 11) | 59.3 | CCCACTTTGACATTCATC CA (SEQ ID NO: 12) | 59.3 | 169 |
| chr1:17668437-17668897 | PADI4 | | CTGTGGTGTTCCAAGACA GC (SEQ ID NO: 13) | 59.3 | GGTAGTGAGAGGGCCAC TTG (SEQ ID NO: 14) | 59.7 | 151 |
| chr2:228356262-228389631 | AGFG1 | hsa_circ_0058514 | TCCCCAGTTGTAGGTCGT TC (SEQ ID NO: 15) | 60 | TAATCCTCGCCTGCATGA CT (SEQ ID NO: 16) | 60.8 | 164 |
| chr4:178274461-178274882 | NEIL3 | hsa_circ_0001459 | TGGATATTCTAACAGATTC AGTGCTC (SEQ ID NO: 17) | 60.1 | [?] CTTGACTTCTGTATGAGG TTTTCC (SEQ ID NO: 18) | 60 | 111 |

The divergent primers have been designed in such a way that they are located on the committed exons in the junction of the ends of the circRNA and oriented towards opposite sides so that they are only amplified in the case that the molecule is circular.

The inventors ran each sample in triplicate (using 10 ng of cDNA in 10 µl of total reaction volume). The q-PCR conditions were: 50°C for 2 minutes, 95°C for 10 minutes, 40 cycles at 95°C for 15 seconds and 60°C for 1 minute followed by a dissociation curve analysis. The raw Cq values and the dissociation curves were analysed in CFX Maestro 1.0 (BioRad), wherein a single peak on the dissociation curve indicates the specificity of the amplification. The change in the expression level of the circRNAs, represented as FC, was calculated in Microsoft Excel 2010 using the 2_DDCq method. Graphpad Prism 6.01 was used for the Student's t test and SPSS for the ROC curves.

### RESULTS

### Identification and characterisation of the circRNA profile in MS

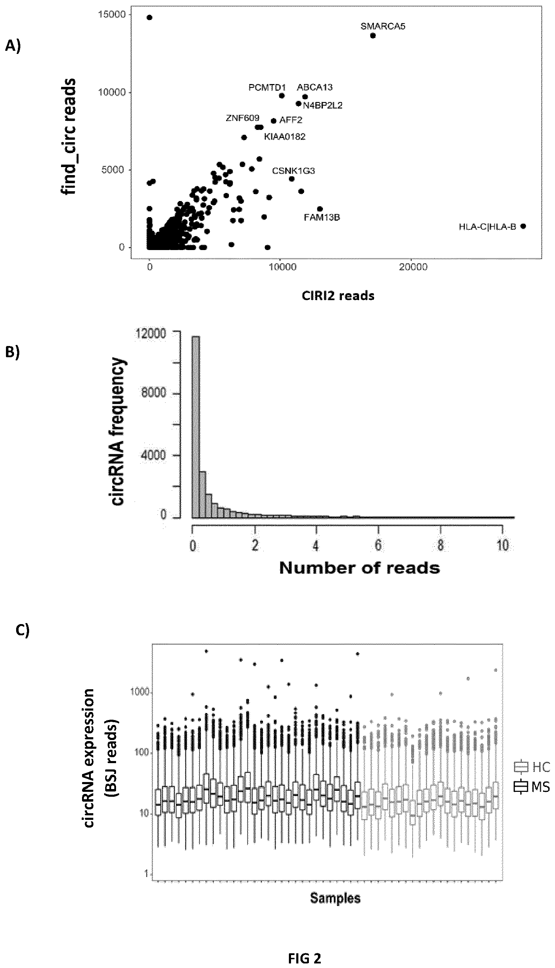

In order to describe the expression profile of circRNA molecules in MS without bias and covering the entire genome, the inventors performed an RNA-Seq of the ribosomal RNA-depleted total RNA from blood samples of patients diagnosed with MS (n = 30) and healthy controls (n = 20). Using Find_circ and CIRI2, they detected a total of 65,726 and 31,314 circRNAs supported by at least two back-spliced junction (BSJ) expansion reads, respectively. They observed good overlap between the two algorithms with 22,835 circRNAs detected in common and with good correlation in read count (Figure 2A).

The expression levels of most circRNAs are low, 96.5% of the circRNAs have been detected with less than 10 reads; however, there is also a small group of 92 circRNAs (0.4%) that are highly abundant and are detected with more than 50 reads (Figure 2B). The distribution of the BSJ read counts of all the samples after standardisation showed that there are no outliers in the data set (Figure 2C).

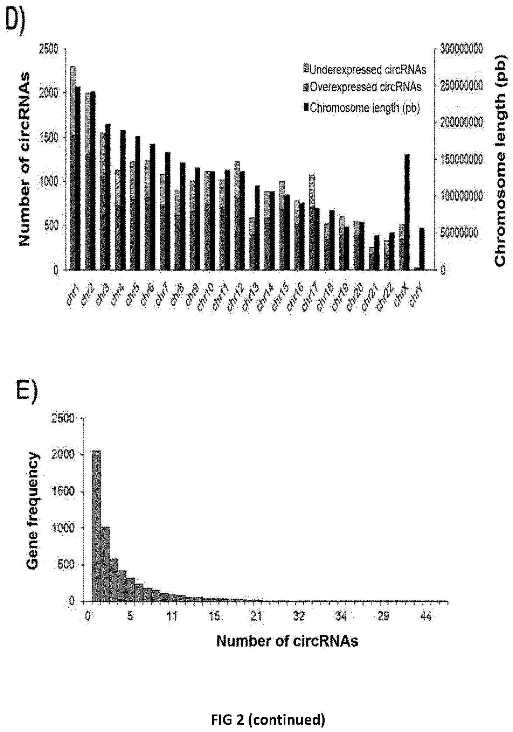

To analyse whether there was any bias in the distribution of the circRNAs along the chromosomes, the number of circRNAs on each chromosome was shown. The number of circRNAs detected was generally consistent with the size of the chromosome for all autosomes, whereas less circRNAs are produced from sex chromosomes, and particularly from the Y chromosome, of which only 19 circRNAs were detected. The proportion of downregulated circRNAs and upregulated circRNAs is also maintained for each of the chromosomes that represent approximately 26-33% and 66-73% of the total number of circRNAs, respectively (Figure 2D).

Of the 22,835 circRNAs detected, 22,197 are within a known gene locus, whereas 638 circRNAs are intergenic. Curiously, only 5,599 different genes have been identified within the genetic regions that produce circRNA, which reveals that each of the genes gives rise to several circRNAs. In fact, 63.3% of genes produce multiple circRNAs with a mean of 5.6 circRNAs produced by each gene. However, it is worth noting that 2,053 genes give rise to a single circRNA and that, at the other end, there are also 533 genes that produce more than ten circRNAs (Figure 2E).

### Circular RNAs are more abundant in patients with MS compared to healthy controls; moreover, a different expression is found depending on gender

Among the 22,835 circRNAs detected in total, only 60% were detected both in the groups with MS and in the healthy controls (HC), whereas some of them were exclusive to one of the groups. It is worth noting that more circRNAs were detected in patients with MS (19,781 circRNAs) compared to healthy controls (16,772 circRNAs). Moreover, with respect to the general expression of most of the circRNAs, a difference between the groups can be observed, which indicates that the circRNAs are more abundant in patients with MS than in healthy controls (Mean expression of the standardised read count MS = 1.973 ± 10.811; HC = 1.854 ± 7.526; p <0.0001) (Figure 2C).

Accordingly, it was observed that 464 circRNAs were differentially expressed (DE-circRNA) with a p-value <0.05 and FC> 1.5 and a clear trend toward positive regulation with more than 96.1% of upregulated circRNAs in patients (446 upregulated circRNAs versus 18 downregulated circRNAs) (Figure 3A).

To find out whether this positive regulation is influenced by gender, the inventors subdivided the cohort into females, 20 with MS versus 14 healthy, and into males, 10 with MS versus 6 healthy, and they performed a second differential expression analysis. Using the same criteria, the same trend toward positive regulation was observed with 498 significantly upregulated circRNAs and only 14 downregulated circRNAs among patients and healthy controls, in other words, 97.3% upregulated circRNAs when the comparison was limited to females (Figure 3B).

For males in general, fewer circRNAs are dysregulated, with a total of 266 differentially regulated circRNAs among patients and healthy controls. In fact, there are fewer circRNAs (193 upregulated circRNAs versus 73 downregulated circRNAs, in other words, 72.5% upregulated circRNAs), and upregulation is also less significant (Figure 3C). Also, it can be seen from the volcano plot that the ratio of upregulation to downregulation does not change as much in males as it does in females (females = 35.6 versus males = 2.6). Furthermore, only 34 circRNAs are differentially expressed in both females and males, whereas 93.4% and 87.21% of the dysregulated circRNAs, respectively, comprise a gender-specific signature (Figure 3D).

Of the 464 DE-circRNAs among all patients with MS and the controls, 68 were highly reliable circRNAs and, depending on the abundance, changes in expression and statistical significance thereof, seven of these circRNAs were selected as candidates for the subsequent validation thereof (Table 3).

**Table 3. FC values and p-value for the seven candidate circRNAs in both the discovery cohort (RNA-Seq) and the validation cohort (qPCR). Genomic coordinates and parental genes are also shown for each circRNA. Significant values are shown in bold. Abbreviations: HC, Healthy Control; MS, Multiple Sclerosis; FC, Fold Change.**

| | | | **MS vs HC** | | | | **MS vs HC (Females)** | | | | **MS vs HC (Males)** | | | |
|---|---|---|---|---|---|---|---|---|---|---|---|---|---|---|
| | | | RNASeq FC (p-value) | | RT-qPCR FC (p-value) | | RNASeq FC (p-value) | | RT-qPCR FC (p-value) | | RNASeq FC (p-value) | | RT-qPCR FC (p-value) | |
| Genomic location (GRCh37) | **Gene** | **circBase ID** | MS N= 30 | HC N= 20 | MS N= 70 | HC N= 46 | MS N= 23 | HC N= 14 | MS N= 58 | HC N= 28 | MS N= 7 | HC N= 6 | MS N= 12 | HC N= 18 |
| chr15:502943 49-50311173 | ATP8B4 | hsa_circ_ 0141241 | **2.39 (<0.0001)** | | **1.63 (<0.0001)** | | **1.92 (0.0003)** | | **1.58 (0.0003)** | | **4.79 (<0.0001)** | | 1.40 (0.1028) | |
| chr2:2283562 62- 228389631 | AGFG1 | hsa_circ_ 0058514 | **2.10 (<0.0001)** | | **1.70 (<0.0001)** | | **1.84 (0.0050)** | | **1.75 (<0.0001)** | | **3.11 (0.0024)** | | 1.35 (0.1591) | |
| chr1:1766843 7-17668897 | PADI4 | circPADI4 | **2.02 (0.0002)** | | **2.95 (<0.0001)** | | **2.29 (0.0002)** | | **2.17 (<0.0001)** | | 1.43 (0.3392) | | **3.61 (<0.0001)** | |
| chrX:1477434 28- 147744289 | AFF2 | hsa_circ_ 0001947 | **1.78 (<0.0001)** | | **1.67 (<0.0001)** | | **1.70 (<0.0001)** | | **1.68 (<0.0001)** | | **2.02 (0.0006)** | | **1.48 (0.0472)** | |
| chr7:4854172 1-48542148 | ABCA13 | hsa_circ_ 0001707 | **1.69 (<0.0001)** | | **1.80 (<0.0001)** | | **1.69 (<0.0001)** | | **1.62 (0.0002)** | | **1.68 (0.0154)** | | **1.91 (0.0088)** | |
| chr4:1782744 61- 178274882 | NEIL3 | hsa_circ_ 0001459 | **1.51 (0.0006)** | | **1.65 (<0.0001)** | | **1.52 (0.0039)** | | **1.81 (<0.0001)** | | 1.50 (0.0857) | | 1.19 (0.4032) | |

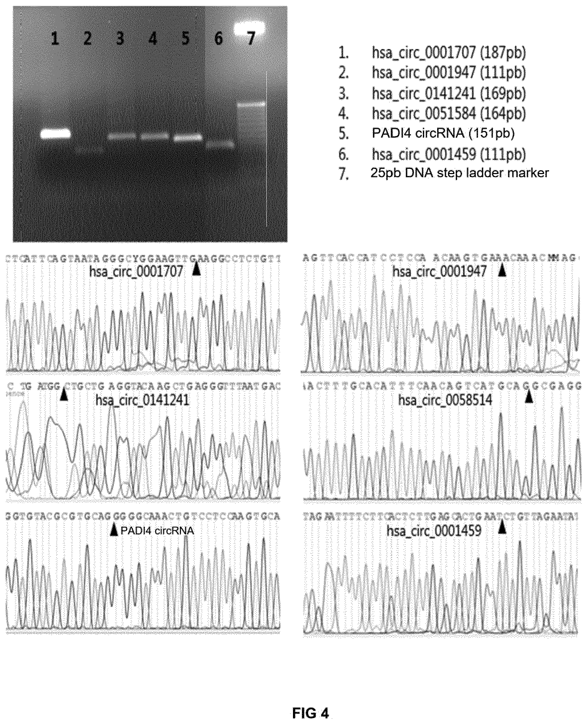

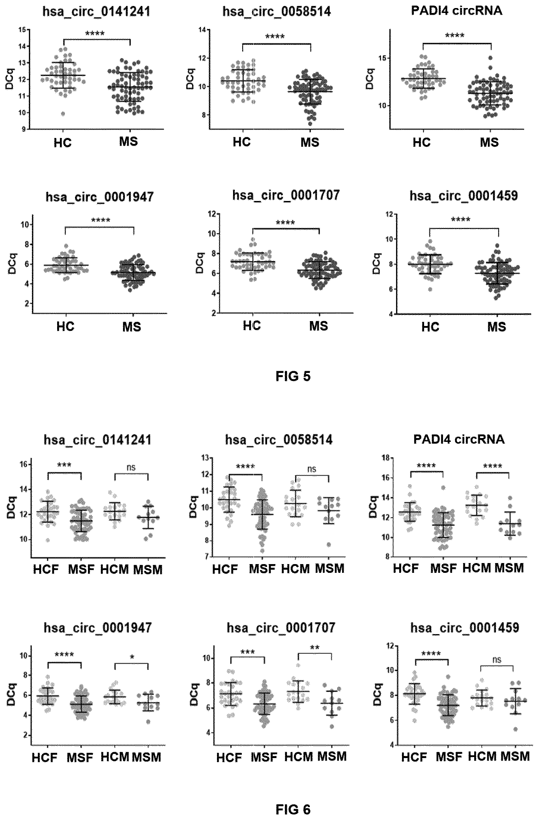

For validation, RT-qPCRs were carried out using divergent primers (Table 2) that span the respective back-spliced junctions for each of the 7 circRNA candidates. Correct amplification of the BSJs was confirmed by agarose gel electrophoresis and Sanger sequencing (Figure 4). Six of the 7 circRNAs were significantly upregulated in a second independent cohort of 70 patients and 46 controls, since although hsa_circ_0001400 also showed a trend of upregulation, this trend was not significant (Figure 5).

Based on the observed gender bias for the RNA-Seq results, and knowing that although all of them are significantly dysregulated throughout the cohort, some of them appear to be gender-specific (Figure 3D). Therefore, circRNA expression was also evaluated separately for females and males in the validation cohort (Figure 6).

The 6 circRNAs that were upregulated were validated for all the samples; they were also significantly upregulated for females. For males, only 3 circRNAs remain significantly upregulated, but it is worth noting that the FC for circPADI4 and hsa_circ_0001707 increases compared to the results obtained for both females and the entire cohort (Table 3).

### CircRNA expression in different types of MS and correlation with clinical characteristics.

Among the 30 patients with MS included in the RNA-Seq experiment, 20 were diagnosed with RR-MS and 10 with SP-MS at the time of blood sampling. The 19,871 circRNAs detected in patients with MS were subjected to a differential expression analysis between both types, resulting in 121 DE-circRNAs (Table 4). These DE-circRNAs represent only 0.61% of the circRNAs detected in patients, which indicates that the general signature of circRNA does not change with the progression of the disease (Figure 7). Moreover, only 7 of those circRNAs were highly reliable.

**Table 4. Differentially expressed circRNAs (FC>|1.5| and p-value <0.05) among patients with SP-MS (n = 10) and RR-MS (n = 20) diagnoses.**

| **Genomic Loc. of the circRNA** | **Gene** | **Mean expression** | **FC (SP-MS vs RR-MS)** | **p-value** |
|---|---|---|---|---|
| chr2:58449076-58459247 | FANCL | 33.03080549 | 1.591620481 | 0.02347373 |
| chr15:101104896-101105470 | | 24.63282674 | 1.892446939 | 0.0268683 |
| chr1:247318867-247323115 | ZNF124 | 18.53146176 | 1.510864326 | 0.00746645 |
| chr18:21644103-21663045 | TTC39C | 13.82291384 | 1.706007921 | 0.00070133 |
| chr1:149239867-149255307 | | 11.70477439 | 1.574408316 | 0.0054596 |
| chr8:618597-624047 | ERICH1 | 11.24479524 | 0.453172417 | 0.03139661 |
| chr1:212502476-212506940 | PPP2R5A | 10.02422956 | 1.587903263 | 0.02112954 |
| chr1 :15860731-15863309 | DNAJC16 | 9.443058549 | 1620182075 | 0.00774876 |
| chr17:16004563-16005119 | NCOR1 | 8.599354199 | 1.578233954 | 0.01086662 |
| chrY:21749095-21749393 | TXLNG2P | 8.359744709 | 10.05520142 | 0.03682505 |
| chr10:70497601-70502326 | CCAR1 | 8.359511589 | 1.543072813 | 0.01978372 |
| chr2:209209834-209212747 | PIKFYVE | 8.238720453 | 0.65171764 | 0.03274322 |
| chr2:68613633-68621308 | PLEK | 7.765333402 | 1.529401339 | 0.02162352 |
| chr6:79752559-79770535 | PHIP | 7.64506656 | 1.571883041 | 0.03447144 |
| chr20:30954186-30959677 | ASXL1 | 7.16460406 | 1.611385137 | 0.01778654 |
| chr12:124904502-124915333 | NCOR2 | 6.960977028 | 1.84895378 | 0.00517348 |
| chr16:68224670-68225678 | NFATC3 | 6.919060569 | 1.660634773 | 0.01472371 |
| chr6:130475969-130505768 | SAMD3 | 6.678618493 | 1.95343164 | 0.03602946 |
| chr11 :102233626-102239279 | BIRC2 | 6.648349979 | 1.524715886 | 0.03600659 |
| chr2:61722589-61726048 | XPO1 | 6.592113665 | 0.55586726 | 0.02721549 |
| chr4:39839475-39843676 | PDS5A | 6.51970463 | 1.815495114 | 0.0138198 |
| chr2:101874252-101879153 | C2orf29 | 6.37078958 | 1.720622977 | 0.00594319 |
| chr7:66456123-66459328 | SBDS | 6.276701622 | 1.702427468 | 0.01415872 |
| chr15:66021409-66031213 | DENND4A | 5.81346098 | 0.45577975 | 0.00142352 |
| chr9:17226200-17236586 | CNTLN | 5.65443237 | 1.845069088 | 0.01360838 |
| chr5:78915434-78919312 | PAPD4 | 5.52014145 | 1.60143642 | 0.03033873 |
| chr10:32832227-32912745 | CCDC7 | 5.381264031 | 0.560516113 | 0.03088615 |
| chr12:32760871-32764217 | FGD4 | 5.371815067 | 0.587501458 | 0.04380741 |
| chr6:90556280-90566918 | CASP8AP2 | 5.350157247 | 1.717481578 | 0.01253552 |
| chr2:8383694-8427688 | LINC00299 | 5.237116226 | 2.484571641 | 0.0333015 |
| chr16:4516153-4519466 | NMRAL1 | 5.162832673 | 1.676390745 | 0.03185248 |
| chr12:112096539-112097149 | BRAP | 5.145608175 | 1.505142825 | 0.04963756 |
| chr6:158994451-159046786 | TMEM181 | 4.977346127 | 2.263265095 | 0.02840198 |
| chr6:76412360-76421132 | SENP6 | 4.865490156 | 1.736349499 | 0.02364353 |
| chr18:46858233-46906128 | DYM | 4.820415227 | 1.726585028 | 0.01472429 |
| chr1:1686812-1687782 | NADK | 4.7617618 | 1.686726742 | 0.01584559 |
| chr5:74130250-74137504 | FAM169A | 4.584573236 | 2.237290803 | 0.02312847 |
| chr1:100960373-100964818 | CDC14A | 4.48655367 | 0.534559773 | 0.04810716 |
| chr12:42778741-42792796 | PPHLN1 | 4.414055199 | 1.92767121 | 0.03308103 |
| chr19:1271327-1272050 | CIRBP | 4.296164667 | 1.978617926 | 0.02694969 |
| chr10:35349801-35360267 | CUL2 | 4.267171421 | 1.961064761 | 0.01758055 |
| chr4:108603170-108615162 | PAPSS1 | 4.156075931 | 1.71305614 | 0.02659178 |
| chr21:40587162-40590586 | BRWD1 | 4.15079965 | 2.433957845 | 0.0148815 |
| chr9:88200377-88248289 | AGTPBP1 | 3.778321278 | 2.055425086 | 0.02232871 |
| chr3:66286967-66313803 | SLC25A26 | 3.58019761 | 2.237657041 | 0.02424046 |
| chr17:40990747-40991397 | PSME3 | 3.544353581 | 2.044522359 | 0.04454345 |
| chr18:42281139-42281797 | SETBP1 | 3.325989838 | 2.502177091 | 0.02288324 |
| chr3:169835013-169840532 | PHC3 | 3.297737789 | 2.528462208 | 0.00968166 |
| chr16:10524467-10525312 | ATF7IP2 | 3.237647456 | 2.271212236 | 0.01557777 |
| chr10:31644072-31676195 | ZEB1 | 3.236162585 | 0.309393138 | 0.00895943 |
| chr2:207144263-207162097 | ZDBF2 | 3.212050318 | 2.695399691 | 0.02059472 |
| chr4:120517677-120528452 | PDE5A | 3.176860619 | 1.96043939 | 0.04341981 |
| chr3:93722502-93722752 | ARL13B | 3.06126987 | 2.224865784 | 0.01227387 |
| chr2:48065997-48066908 | FBXO11 | 2.992449565 | 2.227060517 | 0.01905837 |
| chr3:138400808-138403645 | PIK3CB | 2.842307887 | 0.357224666 | 0.02716552 |
| chr9:99284787-99327765 | CDC14B | 2.68408334 | 0.246862156 | 0.01632605 |
| chr1:24140679-24147083 | HMGCL | 2.653548435 | 2.526775777 | 0.04587771 |
| chr8:56854413-56860282 | LYN | 2.648116207 | 2.764841564 | 0.03206779 |
| chr16:583945-586142 | SOLH | 2.590667953 | 3.706123227 | 0.01594835 |
| chr12:51207792-51208222 | ATF1 | 2.58043415 | 0.274943752 | 0.01322252 |
| chr17:49294694-49302570 | MBTD1 | 2.562247392 | 3.425897879 | 0.00607022 |
| chr11:34107610-34107960 | CAPRIN1 | 2.536861522 | 2.006160038 | 0.03893222 |
| chr3:185638891-185641772 | TRA2B | 2.488913555 | 0.229762733 | 0.0052009 |
| chr2:61528085-61528589 | USP34 | 2.312661511 | 2.544576629 | 0.04620271 |
| chr3:47210658-47210886 | FLJ39534 | 2.218837601 | 0.285748651 | 0.02159106 |
| chr15:41361767-41362745 | INO80 | 2.19720306 | 0.196717537 | 0.00362964 |
| chr20:47708582-47711500 | CSE1L | 2.18414406 | 0.244188516 | 0.02308916 |
| chr3:179481789-179483636 | USP13 | 2.072089236 | 2.893232782 | 0.02383171 |
| chr13:79945079-79946065 | RBM26 | 2.055215083 | 3.520850113 | 0.02340134 |
| chr1:219352488-219385094 | LYPLAL1 | 2.006086531 | 2.658551319 | 0.02283936 |
| chr7:131113791-131130662 | MKLN1 | 1.952821143 | 0.265437532 | 0.03743372 |
| chr19:46116798-46117958 | EML2 | 1.894076653 | 0.271096978 | 0.04534795 |
| chr5:173371969-173380275 | CPEB4 | 1.805567502 | 0.273202468 | 0.03754779 |
| chr10:86223688-86237420 | FAM190B | 1.781594022 | 0.284328844 | 0.0458707 |
| chr2:215617170-215661841 | BARD1 | 1.752593232 | 3.813483603 | 0.02238445 |
| chr10:119078382-119100613 | PDZD8 | 1.725332841 | 3.805311283 | 0.01388618 |
| chr2:173433468-173460751 | PDK1 | 1.721950779 | 3.756864071 | 0.02495339 |
| chr6:84894904-84913814 | KIAA1009 | 1.714510586 | 3.804829108 | 0.0131993 |
| chr18:2762102-2770106 | SMCHD1 | 1.68754888 | 0.06760593 | 8.9744E-05 |
| chr18:18586311-18588155 | ROCK1 | 1.592638832 | 0.216913466 | 0.04441759 |
| chr1:76775190-76779684 | ST6GALNAC3 | 1.472183931 | 0.240350507 | 0.03177726 |
| chr6:144811206-144814592 | UTRN | 1.449869314 | 4.388118522 | 0.02665316 |
| chr11:60899891-60906307 | VPS37C | 1.442514032 | 0.223056205 | 0.02954425 |
| chr7:38284783-38289173 | TRGC2 | 1.437133074 | 5.381639267 | 0.02506204 |
| chr11:72695132-72726930 | FCHSD2 | 1.42981508 | 0.186917031 | 0.04390405 |
| chr5:172535626-172550205 | C5orf41 | 1.38007031 | 0.171103198 | 0.01390125 |
| chr8:81937454-81942324 | PAG1 | 1.369069565 | 0.188286871 | 0.02371591 |
| chr1:114450630-114450813 | DCLRE1B | 1.361516551 | 0.204250051 | 0.04155938 |
| chr8:133634856-133673873 | LRRC6 | 1.35670388 | 0.131478083 | 0.00871469 |
| chr15:52876941-52885933 | FAM214A | 1.33072832 | 0.211675138 | 0.03671858 |
| chr20:48561918-48565892 | RNF114 | 1.307283562 | 0.169313009 | 0.04248233 |
| chr3:129585852-129599405 | TMCC1 | 1.289945088 | 3.886912498 | 0.0371841 |
| chr1:207532890-207536124 | CD55 | 1.261389137 | 0.1645403 | 0.04282777 |
| chr17:66426136-66430767 | WIPI1 | 1.231044499 | 0.145840769 | 0.02151856 |
| chr6:116966876-116982009 | ZUFSP | 1.194575458 | 4.263131099 | 0.02676127 |
| chr2:180837919-180838529 | CWC22 | 1.183276892 | 6.442178698 | 0.0071495 |
| chr11:61094250-61097546 | DDB1 | 1.142221017 | 5.071632923 | 0.01375869 |
| chr14:64988204-64990120 | ZBTB1 | 1.129335875 | 4.659921431 | 0.03501817 |
| chr17:29123177-29131126 | CRLF3 | 1.079125722 | 0.095410729 | 0.00424862 |
| chr9:88574707-88611492 | NAA35 | 1.070380015 | 4.809902291 | 0.03698375 |
| chr2:190656515-190670480 | PMS1 | 1.049310646 | 6.720894017 | 0.01240481 |
| chr14:31103152-31112611 | SCFD1 | 1.022836019 | 7.190760594 | 0.00284738 |
| chr8:37720254-37735069 | RAB11FIP1 | 1.013150762 | 0.195335486 | 0.04907622 |
| chr11:93429485-93433352 | KIAA1731 | 1.000842892 | 7.286272179 | 0.00733659 |
| chr2:197990104-197990761 | ANKRD44 | 0.932879268 | 5.897516302 | 0.04201467 |
| chr6:24869321-24873187 | FAM65B | 0.92608236 | 4.585492266 | 0.03902988 |
| chr2:198281464-198285266 | SF3B1 | 0.892602711 | 0.167137225 | 0.04735349 |
| chr12:72312230-72315234 | TBC1D15 | 0.875680032 | 0.099788848 | 0.01500176 |
| chr3:142122515-142133133 | XRN1 | 0.838447695 | 5.320676571 | 0.04771052 |
| chr7:102960055-102963241 | DNAJC2 | 0.802446209 | 5.448829967 | 0.03894305 |
| chr11:95546095-95552068 | CEP57 | 0.790521514 | 6.12051539 | 0.04179589 |
| chr20:34446223-34457473 | PHF20 | 0.764445863 | 6.727724916 | 0.02440322 |
| chr1:245165422-245222760 | EFCAB2 | 0.698214208 | 0.121095139 | 0.03502719 |
| chr22:47188416-47193517 | TBC1D22A | 0.682941288 | 7.983260572 | 0.04047762 |
| chr20:30671696-30672346 | HCK | 0.663888245 | 11.1118758 | 0.00386272 |
| chr1:161514490-161594429 | FCGR3A | 0.197894363 | 1828797.306 | 2.4807E-07 |
| chr13:64564228-64608670 | AK057471 | 0.197894363 | 1828797.306 | 2.4807E-07 |
| chr7:80275403-80303463 | CD36 | 0.195486132 | 2.44996E-05 | 0.00015165 |
| chr2:219423220-219427536 | USP37 | 0.178513962 | 177950.1348 | 1.5841E-05 |
| chr12:14659095-14689644 | PLBD1 | 0.175906101 | 2018408.69 | 2.0575E-07 |
| chr6:156480809-156481403 | | 0.161490184 | 2.34367E-05 | 0.00014227 |

### CircRNA as biomarkers for the diagnosis of MS

To explore the diagnostic potential of the 6 candidate circRNAs, the overexpression of which had been validated by RT-qPCR, the inventors analysed the Receiver Operating Characteristic curve (ROC Curve). As shown in Figure 8 and Table 5, the 6 circRNAs showed good performance with an area under the curve (AUC) capable of distinguishing patients from healthy controls, wherein the PADI4 circRNA stands out as the circRNA with the greatest diagnostic potential.

Finally, the inventors analysed the possibility of combining several circRNAs to obtain an improvement in the predictive ability of the combination. They observed that the combination of CircRNA_PADI4with the other 5 circRNAs improves the values obtained, which implies that the entire signature of the 6 upregulated circRNAs could have the potential to diagnose 85.2% of the cases of MS.

**Table 5. Area under the curve (AUC) results of the 6 candidate circRNAs and the two proposed combinations.**

| **circRNA** | **Area under the curve** |
|---|---|
| hsa_circ_0001707 | 0.748 |
| hsa_circ_0001947 | 0.753 |
| hsa_circ_0058514 | 0.733 |
| hsa_circ_0141241 | 0.722 |
| hsa_circ_0001459 | 0.740 |
| PADI4 circRNA | 0.843 |
| Combined probability of hsa_circ_17715, hsa_circ_0001707 and hsa_circ_0001459 | 0.847 |
| Combined probability of the 6 circRNAs | 0.853 |

## Claims

1. *In vitro* use of a circRNA comprising the sequence SEQ ID NO: 1 as a biomarker for the diagnosis of multiple sclerosis (MS).

2. The use according to claim 1, wherein the circRNA comprising the sequence SEQ ID NO: 1 is used in combination with at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6.

3. The use according to claim 1 or 2, wherein the circRNA comprising the sequence SEQ ID NO: 1 is used in combination with the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6.

4. An *in vitro* method for the diagnosis of multiple sclerosis comprising:
(a) determining the expression of the circRNA comprising the sequence SEQ ID NO: 1 in an isolated sample from a subject and
(b) comparing it with a reference value, wherein an increase in the expression of said circRNA relative to the reference value indicates that the subject suffers from MS.

5. The *in vitro* method according to claim 4, which further comprises determining the expression of at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6, wherein an increase in the expression of said circRNA relative to the reference value indicates that the subject suffers from MS.

6. The *in vitro* method according to claim 4 or 5, which further comprises determining the expression of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6.

7. The *in vitro* method according to any one of claims 4 to 6, wherein the isolated sample is blood.

8. The *in vitro* method according to any one of claims 4 to 7, wherein the determination of the expression of the circRNA is determined by means of RNA-Seq or real-time PCR (qPCR).

9. The *in vitro* method according to any one of claims 4 to 8, wherein the subject is a human.

10. Use of a kit comprising reagents to quantify the expression of the circRNA comprising the sequence SEQ ID NO: 1, in the method of claim 4, for the diagnosis of MS

11. Use of the kit according to claim 10, which further comprises the reagents to quantify the expression of at least one, two, three, four or five of the circRNAs selected from the list consisting of: the circRNA comprising the sequence SEQ ID NO: 2, the circRNA comprising the sequence SEQ ID NO: 3, the circRNA comprising the sequence SEQ ID NO: 4, the circRNA comprising the sequence SEQ ID NO: 5 and the circRNA comprising the sequence SEQ ID NO: 6.

## Patentansprüche

1. *In-vitro*-Verwendung einer circRNA, die die Sequenz SEQ-ID-NO: 1 als Biomarker für die Diagnose von Multipler Sklerose (MS) umfasst.

2. Verwendung nach Anspruch 1, wobei die circRNA, die die Sequenz SEQ-ID-NO: 1 umfasst, in Kombination mit mindestens einer, zwei, drei, vier oder fünf der circRNA verwendet wird, die aus der Liste ausgewählt sind, die aus Folgendem besteht: der circRNA, die die Sequenz SEQ-ID-NO: 2 umfasst, der circRNA, die die Sequenz SEQ-ID-NO: 3 umfasst, der circRNA, die die Sequenz SEQ-ID-NO: 4 umfasst, der circRNA, die die Sequenz SEQ-ID-NO: 5 umfasst, und der circRNA, die die Sequenz SEQ-ID-NO: 6 umfasst.

3. Verwendung nach Anspruch 1 oder 2, wobei die circRNA, die die Sequenz SEQ-ID-NO: 1 umfasst, in Kombination mit den circRNA verwendet wird, die aus der Liste ausgewählt sind, die aus Folgendem besteht: der circRNA, die die Sequenz SEQ-ID-NO: 2 umfasst, der circRNA, die die Sequenz SEQ-ID-NO: 3 umfasst, der circRNA, die die Sequenz SEQ-ID-NO: 4 umfasst, der circRNA, die die Sequenz SEQ-ID-NO: 5 umfasst, und der circRNA, die die Sequenz SEQ-ID-NO: 6 umfasst.

4. *In-vitro-Verfahren* zur Diagnose von Multipler Sklerose, das Folgendes umfasst:
(a) Bestimmen der Expression der circRNA, die die Sequenz SEQ-ID-NO: 1 umfasst, in einer isolierten Probe von einem Subjekt und
(b) Vergleichen mit einem Referenzwert, wobei ein Anstieg der Expression der circRNA relativ zum Referenzwert anzeigt, dass das Subjekt an MS leidet.

5. *In-vitro-*Verfahren nach Anspruch 4, das ferner das Bestimmen der Expression von mindestens einer, zwei, drei, vier oder fünf der circRNA umfasst, die aus der Liste ausgewählt sind, die aus Folgendem besteht: der circRNA, die die Sequenz SEQ-ID-NO: 2 umfasst, der circRNA, die die Sequenz SEQ-ID-NO: 3 umfasst, der circRNA, die die Sequenz SEQ-ID-NO: 4 umfasst, der circRNA, die die Sequenz SEQ-ID-NO: 5 umfasst, und der circRNA, die die Sequenz SEQ-ID-NO: 6 umfasst, wobei ein Anstieg der Expression der circRNA relativ zum Referenzwert anzeigt, dass das Subjekt an MS leidet.

6. *In-vitro-*Verfahren nach Anspruch 4 oder 5, das ferner das Bestimmen der Expression der circRNA umfasst, die aus der Liste ausgewählt sind, die aus Folgendem besteht: der circRNA, die die Sequenz SEQ-ID-NO: 2 umfasst, der circRNA, die die Sequenz SEQ-ID-NO: 3 umfasst, der circRNA, die die Sequenz SEQ-ID-NO: 4 umfasst, der circRNA, die die Sequenz SEQ-ID-NO: 5 umfasst, und der circRNA, die die Sequenz SEQ-ID-NO: 6 umfasst.

7. *In-vitro-*Verfahren nach einem der Ansprüche 4 bis 6, wobei die isolierte Probe Blut ist.

8. *In-vitro-*Verfahren nach einem der Ansprüche 4 bis 7, wobei die Bestimmung der Expression der circRNA mittels RNA-Seq oder Echtzeit-PCR (qPCR) erfolgt.

9. *In-vitro*-Verfahren nach einem der Ansprüche 4 bis 8, wobei das Subjekt ein Mensch ist.

10. Verwendung eines Kits, das Reagenzien zur Quantifizierung der Expression der circRNA, die die Sequenz SEQ-ID-NO: 1 umfasst, bei dem Verfahren nach Anspruch 4 zur Diagnose von MS umfasst.

11. Verwendung des Kits nach Anspruch 10, das ferner die Reagenzien zur Quantifizierung der Expression von mindestens einer, zwei, drei, vier oder fünf der circRNA umfasst, die aus der Liste ausgewählt sind, die aus Folgendem besteht: der circRNA, die die Sequenz SEQ-ID-NO: 2 umfasst, der circRNA, die die Sequenz SEQ-ID-NO: 3 umfasst, der circRNA, die die Sequenz SEQ-ID-NO: 4 umfasst, der circRNA, die die Sequenz SEQ-ID-NO: 5 umfasst, und der circRNA, die die Sequenz SEQ-ID-NO: 6 umfasst.

## Revendications

1. Utilisation *in vitro* d'un ARNcirc comprenant la séquence SEQ ID NO : 1 en tant que biomarqueur pour le diagnostic de la sclérose en plaques (SEP).

2. Utilisation selon la revendication 1, dans laquelle l'ARNcirc comprenant la séquence SEQ ID NO : 1 est utilisé en combinaison avec au moins un, deux, trois, quatre ou cinq des ARNcirc choisis dans la liste constituée par : l'ARNcirc comprenant la séquence SEQ ID NO : 2, l'ARNcirc comprenant la séquence SEQ ID NO: 3, l'ARNcirc comprenant la séquence SEQ ID NO : 4, l'ARNcirc comprenant la séquence SEQ ID NO : 5 et l'ARNcirc comprenant la séquence SEQ ID NO : 6.

3. Utilisation selon la revendication 1 ou 2, dans laquelle l'ARNcirc comprenant la séquence SEQ ID NO : 1 est utilisé en combinaison avec les ARNcirc choisis dans la liste constituée par : l'ARNcirc comprenant la séquence SEQ ID NO : 2, l'ARNcirc comprenant la séquence SEQ ID NO : 3, l'ARNcirc comprenant la séquence SEQ ID NO : 4, l'ARNcirc comprenant la séquence SEQ ID NO : 5 et l'ARNcirc comprenant la séquence SEQ ID NO : 6.

4. Procédé *in vitro* pour le diagnostic de la sclérose en plaques comprenant :
(a) la détermination de l'expression de l'ARNcirc comprenant la séquence SEQ ID NO : 1 dans un échantillon isolé provenant d'un sujet et
(b) la comparaison de celle-ci à une valeur de référence, dans lequel une augmentation de l'expression dudit ARNcirc par rapport à la valeur de référence indique que le sujet souffre de SEP.

5. Procédé *in vitro* selon la revendication 4, qui comprend en outre la détermination de l'expression d'au moins un, deux, trois, quatre ou cinq des ARNcirc choisis dans la liste constituée par : l'ARNcirc comprenant la séquence SEQ ID NO : 2, l'ARNcirc comprenant la séquence SEQ ID NO : 3, l'ARNcirc comprenant la séquence SEQ ID NO : 4, l'ARNcirc comprenant la séquence SEQ ID NO : 5 et l'ARNcirc comprenant la séquence SEQ ID NO : 6, dans lequel une augmentation de l'expression dudit ARNcirc par rapport à la valeur de référence indique que le sujet souffre de SEP.

6. Procédé *in vitro* selon la revendication 4 ou 5, qui comprend en outre la détermination de l'expression des ARNcirc choisis dans la liste constituée par : l'ARNcirc comprenant la séquence SEQ ID NO : 2, l'ARNcirc comprenant la séquence SEQ ID NO : 3, l'ARNcirc comprenant la séquence SEQ ID NO : 4, l'ARNcirc comprenant la séquence SEQ ID NO : 5 et l'ARNcirc comprenant la séquence SEQ ID NO : 6.

7. Procédé *in vitro* selon l'une quelconque des revendications 4 à 6, dans lequel l'échantillon isolé est du sang.

8. Procédé *in vitro* selon l'une quelconque des revendications 4 à 7, dans lequel la détermination de l'expression de l'ARNcirc est déterminée au moyen d'un séquençage d'ARN ou d'une PCR en temps réel (qPCR).

9. Procédé *in vitro* selon l'une quelconque des revendications 4 à 8, dans lequel le sujet est un être humain.

10. Utilisation d'un kit comprenant des réactifs permettant de quantifier l'expression de l'ARNcirc comprenant la séquence SEQ ID NO: 1, dans le procédé selon la revendication 4, pour le diagnostic de la SEP.

11. Utilisation du kit selon la revendication 10, qui comprend en outre les réactifs permettant de quantifier l'expression d'au moins un, deux, trois, quatre ou cinq des ARNcirc choisis dans la liste constituée par : l'ARNcirc comprenant la séquence SEQ ID NO : 2, l'ARNcirc comprenant la séquence SEQ ID NO : 3, l'ARNcirc comprenant la séquence SEQ ID NO : 4, l'ARNcirc comprenant la séquence SEQ ID NO : 5 et l'ARNcirc comprenant la séquence SEQ ID NO : 6.
